Europäisches Patentamt

European Patent Office

Office européen des brevets

(19)

(11) Publication number: **0 221 282 B1**

## EUROPEAN PATENT SPECIFICATION

(12)

(45) Date of publication of patent specification: **03.07.91**

(51) Int. Cl.⁵: **G01N 33/53**, G01N 33/545, G01N 33/577, A61K 39/395, C12P 21/00

(21) Application number: **86112170.5**

(22) Date of filing: **03.09.86**

The file contains technical information submitted after the application was filed and not included in this specification

(54) **Receptor-antibody sandwich assay.**

(30) Priority: **10.09.85 GB 8522388**

(43) Date of publication of application:
**13.05.87 Bulletin 87/20**

(45) Publication of the grant of the patent:
**03.07.91 Bulletin 91/27**

(84) Designated Contracting States:
**AT BE CH DE FR GB IT LI LU NL SE**

(56) References cited:
**EP-A- 0 101 912      EP-A- 0 122 969**
**EP-A- 0 184 615      US-A- 4 034 074**
**US-A- 4 410 634      US-A- 4 558 009**

*Journal of immunulogy, Vol. 114, no. 4, April 1975, The William & Wilkins Co. (US); B.HEYMER et al., pp. 1191-1195*

(73) Proprietor: **GRUPPO LEPETIT S.p.A.**
**Via G. Murat 23**
**I-20159 Milano(IT)**

(72) Inventor: **Corti, Angelo**
**2, Via Mozzi**
**I-24100 Bergamo(IT)**
Inventor: **Borghi, Angelo**
**36, Via Pierluigi da Palestrina**
**I-20124 Milano(IT)**
Inventor: **Rurali, Carlo**
**19, Viale Vittoria**
**I-20058 Villasanta MI(IT)**
Inventor: **Cassani, Giovanni**
**3, Via Vittadini**
**I-27100 Pavia(IT)**
Inventor: **Parenti, Francesco**
**24, Via Benvenuto Cellini**
**I-20020 LAINATE (MI)(IT)**

(74) Representative: **Sgarbi, Renato et al**
**GRUPPO LEPETIT S.p.A. Patent and Trademark Department 34, Via Roberto Lepetit**
**I-21040 Gerenzano (Varese)(IT)**

## Description

### Prior Art

EP-A-0 184 615, which forms part of the prior art according to Article 54(3) EPC, discloses a solid-phase receptor assay for antibiotics of the vancomycin class.

US-A-4 034 074 discloses a two-site solid-phase immunoradiometric assay using labelled anti-(IgG).

### Disclosure of the present invention

The present invention concerns a new sandwich assay for determining a substance capable of binding to a D-Alanyl-D-Alanine dipeptide or a D-Alanyl-D-Alanine carboxy terminal oligopeptide. It has particular application to determining a glycopeptidic antibiotic of the vancomycin class or a derivative or aglycon thereof. The assay combines the high selectivity of a suitable D-Alanyl-D-Alanine derivative for the antibiotics of the vancomycin class and the specificity of an antibody directed against the antibiotic of the class which is the object of the determination.

Examples of antibiotics of the vancomycin class are (in addition to vancomycin): teicoplanin, actaplanin, ristocetin, avoparcin, actinoidin, antibiotic LL-AM-374, antibiotic A 477, antibiotic OA 7653, antibiotic A 35512 B, antibiotic A 51568, antibiotic AAD 216, antibiotic A 41030, antibiotic A 47934 as well as the individual factors, derivatives and aglycons thereof.

It is known that vancomycin and ristocetin A interfere with the synthesis of peptidoglycan, which is the main structural component of the bacterial cell-wall. It is thought that this interference, which leads to the inhibition of cell growth and, eventually, to the destruction of the cell by lysis, is due to a specific binding between these antibiotics and the pentapeptide precursor having a D-Ala-D-Ala residue at the carboxyl terminus (UDP-N-Acetylmuramylpentapeptide) (H.R. Perkins, Biochem. J. 111, 195, (1969)).

It was recently discovered that also teicoplanin acts through the same mechanism by binding to the growing peptidoglycan of sensitive bacteria. More particularly, it was found that teicoplanin, like vancomycin and ristocetin A and similar antibiotics, binds to peptidoglycans containing a D-Ala-D-Ala dipeptide at the carboxy terminus. By so doing, they probably inhibit the bacterial transpeptidase and prevent the cross-linking of cell-wall peptidoglycan. These antibiotics also bind a D-Alanyl-D-Alanine dipeptide or a D-Alanyl-D-Alanine carboxy terminal oligopeptide. Also the other antibiotics of the vancomycin class have proven or are supposed to act through the same mechanism of action, and are therefore capable of binding to a D-Alanyl-D-Alanine dipeptide or a D-Alanyl-D-Alanine carboxy terminal oligopeptide and can elicit the formation of antibodies against them in a suitable host.

In addition to the antibiotics of the vancomycin class, the present method can suitably be used for determining other non-glycopeptidic or non-vancomycin-like antibiotics which are capable of binding to a D-Alanyl-D-Alanine dipeptide or a D-Alanyl-D-Alanine carboxy terminal oligopeptide and that can elicit the formation of antibodies against them in a suitable host.

D-Alanyl-D-Alanine or a D-Alanyl-D-Alanine oligopeptide can be considered therefore as a "molecular receptor" for the above described antibiotic substances.

Methods known so far for determining antibiotic substances and in particular teicoplanin and the other antibiotics of the vancomycin class are mainly based on TLC, HPLC, and bioassays on susceptible microorganisms. In view of the current therapeutic use or advanced clinical study of some of these antibiotics, there is a need for assay methods for their determination in fluids, especially biological fluids, which be specific, rapid, easy, reliable, and suitable for automation.

In particular, the detection of these substances in e.g. body exudates, bronchial expectorates, pus and skin samples from burned patients is particularly difficult with known techniques since false-positive results are often obtained. The method of the invention, on the contrary, is accurate and reliable also on these samples.

The method of the invention is therefore a rapid, easy, reliable, direct method for specifically determining an analyte which possesses the feature of binding to the "molecular receptor" represented by a D-Alanyl-D-Alanine dipeptide or a D-Alanyl-D-Alanine carboxy terminal oligopeptide. In particular, the method of this invention allows the specific determination of an antibiotic of the vancomycin class even in the presence of another antibiotic of the same class with high sensitivity, accuracy and precision.

More particularly, the method of the invention includes:

a) causing a test solution to contact a surface supporting a D-Alanyl-D-Alanine carboxy terminal oligopeptide conjugated with a suitable macromolecular carrier capable of adsorbing onto said surface;

b) after rinsing, adding antibodies specifically directed against the substance to be detected (analyte);

c) revealing said antibodies bound to the antigen on the stationary phase by means of a species-specific anti-IgG-antibody directed toward these bound antibodies (anti-antibody antiserum) coupled to a detectable marker.

The D-Alanyl-D-Alanine carboxy terminal oligopeptide may be a tri-, tetra-, penta-, hexa-, or heptapeptide wherein the carboxy terminal dipeptide is represented by D-Alanyl-D-Alanine. The preferred D-Alanyl-D-Alanine carboxy terminal oligopeptide of the invention is the tripeptide Epsilon-aminocaproyl-D-Alanyl-D-Alanine (hereinafter: Epsilon-Aca-D-Ala-D-Ala).

The D-Alanyl-D-Alanine carboxy terminal oligopeptide is conjugated with a suitable carrier in order to make it adsorbable on a solid phase surface. The suitable carrier may be in this case any protein or other high molecular weight substance capable of being covalently linked to the D-Alanyl-D-Alanine carboxy terminal oligopeptide to give a conjugate which is capable of adsorbing (i.e. capable of forming physical bonds) with the selected solid phase surface.

A preferred example of such a carrier is albumin, and bovine albumin in particular. However egg, human or pork albumin are usable as well.

The solid phase surface onto which the D-Alanyl-D-Alanine carboxy terminal oligopeptide-bearing carrier is adsorbed is preferably the surface of an analytical support. In the present application, the term "analytical support" refers to any analytical means which can contain or, at least, be exposed to liquid reagents and can therefore be used as a surface supporting a D-Alanyl-D-Alanine carboxy terminal oligopeptide conjugated with a suitable macromolecular carrier. Preferred examples of these are glass or plastic microtiter plates or test tubes or plastic sheets. The most preferred "analytical support" is represented by the wells of a polyethylene or polystyrene microtiter plate.

Accordingly, the most preferred "coated analytical support" (i.e. surface having the oligopeptide supported thereon) of the invention is plastic wells or tubes coated with from 0.025 microgram to 1 microgram preferably from 0.1 to 0.2 microgram of BSA-Epsilon-Aca-D-Ala-D-Ala.

As already stated, the analyte supposed to be present in the "test solution" is an antibiotic of the vancomycin class or, more generally, a substance capable of binding to a D-Alanyl-D-Alanine dipeptide or a D-Alanyl-D-Alanine carboxy terminal oligopeptide.

A preferred analyte is teicoplanin or an individual factor, derivative or aglycon thereof.

"Antibodies specifically directed to the substance to be determined" are antibodies elicited in an animal by injection of a suitable conjugate of the substance to be tested. These antibodies can be conventional antisera, which are obtained by immunizing an animal such as a rabbit, rat or another non-human mammalian, or can be monoclonal antibodies to said substances prepared essentially following the cell-fusion technique of C. Milstein and G. Köhler (Nature, 256, 495-497 (1975)).

Since the analytes are haptens (i.e. they are not capable of eliciting as such the production of antibodies against them when injected in a non-human mammalian, but have immunogenic properties which can be expressed when they are coupled to a suitable high molecular weight carrier such as a protein), they are conjugated to a suitable carrier, which is in general a protein such as bovine serum albumin, prior to immunizing the selected host animal.

A convenient host animal for producing a conventional antiserum, which can then be purified or fractionated to isolate the IgGs, is the rabbit. On the contrary monoclonal antibodies are conventionally produced by hybridomas obtained by fusion of rat spleen cells and homologous myeloma cells in the presence of a fusion promoter by essentially following the procedure of Milstein and Köhler cited above. For a convenient use in the method of the invention, both a significant proportion of the polyclonal antibodies of the conventional antiserum and the monoclonal antibodies which may represent the "antibodies specifically directed to the substance to be determined" must not interfere with the binding of the analyte (i.e. the antibiotic to be determined) with its "molecular receptor" (i.e. the D-Ala-D-Ala oligopeptide adsorbed on the suitable surface) and obviously must not cross-react with the other antibiotics of the class that may be present in the sample and simultaneously bind to the molecular receptor.

The "anti-antibody antisera" used in the method of the invention are conventional antibody preparations against the immunoglobulins G (IgGs) of a certain animal species, which is the animal species to which the "first" antibody (i.e. that directed against the analyte) belongs. Preparations of these antibodies, such as goat-antiserum to rabbit, goat-anti-rat antibodies, are obtained according to conventional techniques and are also commercially available.

The detectable marker which is coupled with this anti-antibody antiserum is one of the usual "markers" in immunoassays such as an enzyme, a radioisotope or a fluorescent dye. A preferred enzyme is a peroxidase, such as horseradish peroxidase, which is revealed and quantitatively determined colorimetrically by means of one of its chromogenic substrates such as orthophenylendiamine in the presence of hydrogen peroxide. Also the anti-antibody antisera coupled to the detectable marker are commercially available or can be prepared by conventional procedures.

As will be appreciated by those skilled in the art, the coupling of the D-Alanyl-D-Alanine carboxy terminal oligopeptide with the suitable carrier may be obtained by means of techniques known per se . Conveniently, a coupling agent is employed; a preferred coupling agent being glutaraldehyde. Other known coupling agents include: carbodiimides, diisocyanates, anhydrides, diazonium compounds, azides, and cyanogen bromide. Similarly, known coupling procedures and reagents may be used for coupling the anti-antibody antiserum to the detectable marker. These procedures depend mainly on the characteristics of the selected detectable marker. When the detectable marker is an enzyme, coupling procedures like those reported above can be used. Also in this case, glutaraldehyde is the preferred coupling agent. In these coupling reactions, the proportion between the reactants may vary significantly, even if in many instances a ratio of about 1:1 between the reagents is preferred. Moreover, in the case of the coupling reaction between a D-Alanyl-D-Alanine carboxy terminal oligopeptide which is Epsilon-Aca-D-Ala-D-Ala and a macromolecular carrier which is bovine serum albumin (BSA) the molar proportion between these two reagents may vary from 1:1 to 10:1.

Described in more detail, a preferred embodiment of the method of the invention is the following:

a) preparing a standard curve with known amounts of the analyte in the suitable dilution buffer,

b) preparing the samples and suitably diluting them in the same dilution buffer as above,

c) adding, separately, the standard dilution and the samples to the coated analytical support defined above,

d) after incubating at room temperature in a humid covered box, washing the coated analytical support and adding a preparation of the "first" antibody (anti-analyte antibody) conveniently diluted in the above dilution buffer,

e) after washing again the coated analytical support and drying, adding the "second" antibody preparation (anti-antibody preparation) wherein the species-specific antibody is coupled to a detectable marker, and finally

f) by determining said "detectable marker" bound to the coated analytical system, evaluating the concentration of the analyte in the sample by reference to the values of the standard curve.

The above dilution buffer is an aqueous buffer and preferably phosphate buffered saline at a pH between 7.0 and 7.5. Preferably, when the sample is serum, bronchial expectorates, pus or skin, the dilution buffer contains from 10% to 30% (by volume) and most preferably 20% of serum or a serum protein preparation.

An advantage of the method of the invention is that it is now possible to easily and rapidly determine an antibiotic of the vancomycin class in the presence of another member of the same class. While in fact all the antibiotics of the vancomycin class are linked selectively, although to a different extent, to a D-Ala-D-Ala carboxy terminal peptide, only the antibiotic to be tested will react with the specific antibody. Therefore when assaying a biological fluid which contains for instance two antibiotics of the vancomycin class (e.g. vancomycin and teicoplanin) by means of the method of the invention, both antibiotics will be selectively bound to the analytical support having adsorbed onto the surface a D-Alanyl-D-Alanine carboxy terminal oligopeptide conjugated with the suitable macromolecular carrier, while all the other substances which are present in the sample and usually do interfere will be washed away. Then the specific antibody (e.g. an antibody directed against teicoplanin) will bind only to the relevant antibiotic (e.g. teicoplanin) bound to the adsorbed D-Alanyl-D-Alanine peptide but not to the similarly bound other antibiotic (e.g. vancomycin).

The species-specific anti-antibody antiserum coupled with the detectable marker, once added, will therefore specifically bind to the anti-analyte (in this case, anti-teicoplanin) antibody which is, in turn, bound to the "stationary phase" through the binding of the specific antigen (in this case, teicoplanin). The subsequent evaluation of the "bound" species-specific anti-antibody antiserum by means of the detection of the coupled "marker" is therefore correlated only to the specific antibiotic of the class which is the selected object of the assay (in this case, teicoplanin) even in the presence of other antibiotics of the same class (in this case vancomycin).

A preferred embodiment of the method of the invention is a method as described above wherein the analytical support bearing on the surface a D-Alanyl-D-Alanine carboxy terminal oligopeptide conjugated with a suitable macromolecular carrier capable of adsorbing onto said surface is the wells of a plastic microtitre plate coated with BSA-Epsilon-Aca-D-Ala-D-Ala, the antibodies directed to the substance to be detected are rabbit antisera against an antibiotic of the vancomycin class and the anti-antibody antiserum coupled with a detectable marker is goat-antiserum to rabbit IgG coupled with a peroxidase.

The rabbit antisera against a predetermined antibiotic of the vancomycin class ("predetermined antibiotic") are obtained by repeatedly injecting (e.g. every three weeks for three months) into rabbits effective anti-"predetermined antibiotic" antibody eliciting amounts of a "predetermined antibiotic" -albumin conjugate prepared by coupling the "predetermined antibiotic" and bovine serum albumin by means of methods known per se by using, for instance, from about one to about ten parts by weight of "predetermined-antibiotic" per part by weight of bovine serum albumin.

The animals are bled weekly and anti-"predetermined antibiotic" antibody titres are determined by analytical method, for instance, an ELISA method using an analytical support coated with the same "predetermined antibiotic" and an appropriate detectable probe, e.g. goat-antiserum to rabbit IgGs coupled with peroxidase. When analytically effective antibody production is achieved, the animals are bled and the antiserum is recovered and then, optionally, submitted to further purification mainly for removal of anti-bovine serum albumin immunoglobulins. Said purification may involve absorption chromatography with a modified matrix, for instance a matrix prepared by coupling bovine serum albumin with an agarose gel (e.g. Sepharose ®) activated by methods known per se. The term "analytical support" as used here has the same meaning as above. In particular, PVC-microtitre plates, test tubes or plastic sheets capable of adsorbing onto the surface analytically detectable amounts of the "predetermined antibiotic", are particularly suitable means for use in the analytical method.

Besides being useful in specifically, rapidly, easily detecting and assaying the analyte in solutions such as those from fermentation broths or extracts, the present method is also applicable for detecting and assaying biological fluids such as blood and its fractions, urine, body exudates, pus, bronchial expectorates and skin.

In order to apply the present method to a biological fluid, in general it is not necessary to perform a particular pre-treatment of the sample but, usually, dilution with a buffer (and, rarely, concentration of the sample) to enter the specific application range of the method under the selected conditions will be sufficient.

Preferably, the calibration curve is obtained with a standard preparation of the analyte dissolved in the same biological fluid of the sample to be assayed.

In the case of bronchial expectorates, exudates, pus and skin it is in general necessary to homogenize the sample before the analysis by usual means. The sample is in general diluted with a buffer, normally before homogenization.

Preferably, the buffer is an aqueous buffer at a pH from 7 to 7.5 with serum or serum proteins added to 10-30% of the total volume, preferably 20% of the total volume of the dilution buffer.

A preferred embodiment of the invention is represented by the use of anti-teicoplanin antiserum which does not cross-react with vancomycin, avoparcin, ristocetin, antibiotic A 35512 B, actaplanin, antibiotic A 41030, antibiotic A 47934 and the other antibiotics of the vancomycin class.

The preferred application range of the present method is from 10 to 1000 ng/ml of analyte; the most preferred range being from 10 to 100 ng/ml. The inter assay precision (CV) is in general between 10-15%.

The accuracy and precision of the method can be further improved by a proper experiment design aimed at minimizing the errors with the contribution of the known statistical distribution methods.

The "analytical support" of the invention is suitable for use in manual as well as automated assay procedures. This flexibility is of particular importance to meet the needs of the final user. In fact, an automated procedure may be extremely useful in clinical monitoring of many treated patients e.g. for controlling the therapeutic dose and studying the drug distribution into the biological fluids, while a manual procedure may be sufficient for lab-scale analysis.

In its composition of matter aspect, the present invention encompasses also a kit of reagents for exploiting the method of the invention for the easy, rapid, reliable, and accurate determination of an analyte which possesses the feature of binding to the "molecular receptor" represented by a D-Alanyl-D-Alanine dipeptide or a D-Alanyl-D-Alanine carboxy terminal oligopeptide, especially, an antibiotic of the vancomycin class, an individual factor, derivative or aglycon thereof, which includes:

a) an analytical support having a surface supporting a D-Alanyl-D-Alanine carboxy terminal oligopeptide conjugated with a suitable macromolecular carrier capable of adsorbing onto said surface;

b) an antibody preparation containing antibodies specifically directed against the analyte; and optionally

c) a species-specific anti-antibody preparation which contains antibodies specifically directed against the immunoglobulins of the animal species of origin of the "first" antibody (which is that directed against the analyte).

As already stated, a preferred "analytical support having a surface supporting a D-Alanyl-D-Alanine carboxy terminal oligopeptide conjugated with a suitable macromolecular carrier capable of adsorbing onto said surface" (coated analytical support) is represented by plastic wells of plates for microtitrations, test tubes or plastic sheets coated with from 0.025 microgram to 1 microgram, and preferably from 0.1 to 0.2 microgram of BSA-Epsilon-Aca-D-Ala-D-Ala.

The amount of antibody specific for the analyte in the first antibody preparation must exceed the amount of analyte to be detected. It is represented by a monoclonal antibody or by a polyclonal antibody preferably obtained by immunized rabbits having an antibody titer higher than $10^{-4}$ at the time of bleeding.

Evidently, the concentration of the "second" antibody preparation, i.e. the species-specific antibody preparation, is related to the concentration of the "first" antibody which is going to be bound to the analyte on the supporting solid surface and should be in an equimolar proportion or in a convenient excess.

It will be evident to the man skilled in the art that not only other components may optionally be present in the "kit" such as buffer solutions, revealing means, etc., but also some of the components of the kit may be presented in a separate form but for the same use. Also these separate forms of components for use in the present method are encompassed by the composition of matter and method of the present invention.

The invention further provides an anti-teicoplanin rabbit antiserum, obtained by repeatedly injecting into rabbits an effective anti-teicoplanin antibody eliciting amount of a teicoplanin-albumin conjugate prepared by coupling from one to ten parts by weight of teicoplanin for each part by weight of bovine serum albumin bleeding the animals after a period of time sufficient to achieve an analytically effective antibody production, recovering the obtained antiserum, and optionally purifying it from anti-bovine serum albumin immunoglobulins by contacting with an equal amount (by volume) of a matrix obtained by coupling one part by weight of bovine serum albumin with one hundred parts (by volume) of activated swollen agarose.

The invention additionally provides a coated analytical support for use in analytical determination of teicoplanin and anti-teicoplanin antibodies consisting of a PVC-microtitre plate, test tube or plastic sheet, adsorbing onto the surface a suitable amount of teicoplanin.

The following Examples further illustrate the invention.

Example 1:

Preparation of BSA-Epsilon-Aca-D-Ala-D-Ala

a) Conjugation of Epsilon-aminocaproyl-D-alanyl-D-alanine (Epsilon-Aca-D-Ala-D-Ala) to bovine serum albumin (BSA)

Epsilon-Aca-D-Ala-D-Ala is conjugated to BSA by a "two step method" using glutaraldehyde as the bifunctional cross-linking reagent: BSA (250 mg) is dissolved in 4.5ml 0.1 M Na-carbonate/bicarbonate buffer at pH 9.5. Glutaraldehyde (0.5 ml of a 25% aqueous solution) is added and allowed to react for 1 hour at room temperature. The reaction mass is then dialyzed against 0.9% NaCl overnight. Epsilon-Aca-D-Ala-D-Ala (125 mg) is dissolved in 0.5 ml of 0.5 M Na-carbonate buffer at pH 9.5, added to the dialyzed reaction mass and stirred for 6 h at room temperature. 1 M lysine (0.5 ml) is then added and the mixture is kept at room temperature for 2 h to block the unreacted active groups. The conjugate is then dialyzed against 0.05 M K-phosphate containing 0.15 M NaCl pH 7.3.

b) Gel Filtration of the Conjugate

To purify the conjugate from excess unreacted material or low molecular weight by-products the above obtained dialyzed mixture is passed through a refrigerated Sephacryl ® S-200 column (controlled pore covalently cross-linked alkyl dextrane and N,N'-methylene bis-acrylamide) (Pharmacia Fine Chemicals) (0.8 cm × 100 cm) contacted and eluted with PBS brought to pH 10.4 (flow rate = 10 ml/h). The elution is monitored spectrophotometrically by registering the transmittance at 280 nm. Fractions corresponding to the peak eluted with the void volume of the column are pooled (total volume = 15 ml), neutralized with 1 M HCl and diluted four times with PBS pH 7.3. Aliquots of 0.5 ml of this mixture are distributed into vials, lyophilized and kept at -80° C.

Example 2:

Coating of microplates with BSA-Epsilon-Aca-D-Ala-D-Ala.

A stock solution for the weekly preparation of microtitre plates is prepared by dissolving the content of a vial of lyophilized BSA-Epsilon-Aca-D-Ala-D-Ala (prepared as described in the foregoing Example) in PBS pH 7.3 (0.5 ml); this solution can be stored for weeks without loss of activity and is therefore suitable for use as the stock solution for weekly preparations.

The wells of microtitre plates (Falcon Microtest ® III Flexible Assay Plate; Becton Dickinson and Co., Oxnard, California 93030) are filled with 100 microliter/well of a 1/200 dilution of this stock solution, except for one vertical row of wells (conveniently the last one) which is kept as the blank. These blank wells are filled with 100 microliter of PBS pH 7.3. The microtitre plates are incubated for 3 hours at room temperature in a covered box and then washed 5 times by emptying and filling with distilled water. 200 microliter/well of 3% BSA in PBS pH 7.3 were added and allowed to adsorb for 2 hours at room temperature for blocking uncoated plastic surface. The plates are washed again with distilled water, shaken dry and stored at 4°C until use.

Example 3:

Preparation of anti-teicoplanin antiserum

A teicoplanin-albumin conjugate (T-BSA) is used for immunization of rabbits in order to elicit the production of anti-teicoplanin antibodies.

T-BSA is prepared by coupling 200 mg of teicoplanin to 50 mg of BSA using 1-ethyl-3-(3-methylaminopropyl)-carbodiimide hydrochloride (300 mg) as the coupling reagent in 5 ml of water at pH 5.5 according to Goodfriend et al. (Goodfriend T.L., C. Levine and G.D. Fasman, 1964, Science 144, 1344). The T-BSA is dialyzed overnight, lyophilized and then suspended at a concentration of 1 mg/ml in a physiological solution containing 50% by volume of Freund's adjuvant. Five female healthy New Zealand white rabbits are injected s.c. each with 1.2 ml of the above solution. Booster injections are made to each rabbit with 0.5 ml of the same antigen solution every three weeks for three months. During this period the animals are bled weekly and anti-teicoplanin titres are determined by an ELISA method with teicoplanin coated microtitre plates using horseradish peroxidase labelled goat-antiserum to rabbit IgGs as the revealing probe.

The ELISA method mentioned above is carried out as follows.

PVC-microtitre plates (Falcon Microtest ® III Flexible Assay Plate; Becton Dickinson and Co., Oxnard, California 93030) are filled with 0.1 ml/well of a solution containing 0.4 g/l of teicoplanin and 58 g/l of NaCl. The microtitre plates are incubated for three hours at 37°C in a covered box and then washed three times by emptying and filling with phosphate buffered saline pH 7.3 (0.15 M NaCl, 0.05 M sodium phosphate).

A solution of BSA (30 g/l) in phosphate buffered saline pH 7.3 (0.15 M NaCl, 0.05 M sodium phosphate) is added to wells (0.2 ml/well) and allowed to adsorb for two hours at room temperature to block the uncoated plastic surface. The plates are washed again three times with phosphate buffered saline pH 7.3 (0.15 M NaCl, 0.05 M sodium phosphate) and then rabbit antiserum test solution is added (0.1 ml/well) at different ten-scalar dilutions (from 1:10 to 1:10^6) and allowed to react with the sensitized wells for two hours at 37°C. After thoroughly washing with phosphate buffered saline pH 7.3 (0.15 M NaCl, 0.05 M sodium phosphate) a goat-antiserum to rabbit IgGs labelled with horseradish peroxidase is added to wells (0.1 ml/well) and allowed to react for one hour at 37°C. After thoroughly washing with phosphate buffered saline pH 7.3 (0.05 M sodium phosphate, 0.15 M sodium chloride) containing 0.01% (v/v) Tween ® 20, the microtitre plates are shaken dry and 0.2 ml of the peroxidase substrate chromogenic solution (1 mg/ml o-phenylenediamine, 5 mM hydrogen peroxide in 0.1 M sodium citrate buffer pH 5) added to each microtiter well.

The color is allowed to develop for 30 minutes at room temperature and stopped by adding 0.05 ml/well of 4.5 M sulfuric acid.

After 15 minutes the absorbance at 492 nm is measured. The antibody titre is expressed as the dilution factor giving 0.2 optical density units.

When the antibody titre is at least 1:10^4, analytically effective antibody production is achieved and the animals are completely bled and the desired antiserum is recovered and kept at -20°C until use.

Example 4:

Purification of anti-teicoplanin rabbit antiserum on BSA-agarose

A modified matrix is prepared by coupling bovine serum albumin (BSA), 80 mg, with CNBr-activated Sepharose ® (Pharmacia Fine Chemicals; 10 ml of swollen resin) using 0.1 M sodium carbonate buffer (pH 8.3) as the coupling buffer. This BSA-agarose matrix is then used to remove anti-BSA immunoglobulins from the antiserum obtained according to the foregoing Example 3 by batch adsorption chromatography by contacting 2 ml of said rabbit antiserum with 2 ml of the sedimented BSA-agarose matrix. This mixture is allowed to contact for 1 h at room temperature and then poured onto a glass filter and is washed three times with 6 ml phosphate buffered saline solution pH 7.3 (0.05 M sodium phosphate, 0.15 M sodium chloride) containing 3% (w/v) BSA and 0.05% (v/v) Tween ® 20. The recovered filtrate corresponds to a ten-fold dilution of the original anti-serum from which the anti-BSA antibodies have been removed.

Example 5:

Assay of teicoplanin containing solutions

A standard curve is prepared with scalar dilutions of teicoplanin at concentrations from 0.001 to 1 microgram/ml in phosphate buffered saline pH 7.3 (0.05 M sodium phosphate, 0.15 M sodium chloride) containing 3% (w/v) BSA and 0.05% (v/v) Tween ® 20. 100 μl of these solutions as well as the samples which have been diluted ten-fold in the serum buffer are added, separately, in triplicate to the wells of BSA-Epsilon-Aca-D-Ala-D-Ala coated flexible plates prepared according to the procedure of the foregoing Example 2. The solution with the higher concentration is added also to the "blank" wells (i.e. those which are not coated with the specific D-Ala-D-Ala binder) to evaluate the nonspecific binding. Each microtitre plate is then incubated for 2 h at room temperature in a humid covered box and washed eight times by emptying and filling with phosphate buffered saline solution pH 7.3 (0.05 M sodium phosphate, 0.15 M sodium chloride containing 0.05% (v/v) Tween ® 20) and shaken dry.

Purified rabbit antiserum against teicoplanin prepared according to the foregoing Examples 3 and 4 is then diluted 50 fold with phosphate buffered saline solution (0.05 M sodium phosphate, 0.15 M sodium chloride, pH 7.3 containing 3% (w/v) BSA and 0.05% (v/v) Tween ® 20); 100 microliter of this solution is added to each well and allowed to react for 1 h at room temperature. After washing eight times with phosphate buffered saline pH 7.3 (0.05 M sodium phosphate, 0.15 M sodium chloride containing 0.05% (v/v) Tween ® 20), shaken dry as described above, 100 microliters of a 1 to 500 dilution in phosphate buffered saline of horseradish peroxidase conjugated goat-antiserum to rabbit IgGs (Bionetics Inc.) is added to each well and allowed to stand for 1 h at room temperature. Then each well is again washed with phosphate buffered saline pH 7.3 (0.05 M sodium phosphate, 0.15 M sodium chloride containing 0.05% (v/v) Tween ® 20) and shaken dry as described above and a 200 microliter/well solution of a peroxidase chromogenic substrate (1 mg/ml o-phenylendiamine, 5 mM hydrogen peroxide in 0.1 M sodium citrate buffer, pH 5) is added to each well and the color allowed to develop for 30 min at room temperature. The color developing reaction is then stopped by adding 50 microliter/well of 4.5 M sulfuric acid and, after 15 min, the absorbance at 492 nm is measured in a Titertek Multiscan photometer (Flow Lab. Inc.). Binding curves are obtained by plotting the values of absorbance as a function of the concentration of teicoplanin on a semi-log paper. A dose-response curve is obtained with teicoplanin in the range from 0.01 microgram/ml to 1 microgram/ml and the concentration in the sample is determined by interpolation on the standard curve.

The repeated assay of solutions containing known amounts of teicoplanin by means of the above procedure indicates that this method satisfies the analytical requirements of sensitivity, precision and accuracy.

No binding was observed repeating the above method and using solutions of avoparcin, ristocetin, actaplanin, antibiotic A 35512 B, antibiotic A 41030, antibiotic A 47934 and vancomycin.

Example 6:

Assay of teicoplanin containing serum samples

A standard curve is prepared with scalar dilutions of teicoplanin at concentrations from 0.001 to 1 microgram/ml in phosphate buffered saline pH 7.3 (0.05 M sodium phosphate, 0.15 M sodium chloride) containing 3% (w/v) BSA, 0.05% (v/v) Tween ® 20 and 20% human serum.

The samples are five-fold diluted in the serum buffer and 0.1 ml of these solutions are added in triplicate to the wells of BSA-Epsilon-Aca-D-Ala-D-Ala coated flexible plates prepared according to the procedure of the foregoing Example 2. The solution with the higher concentration is added also to the "blank" wells (i.e. those which are not coated with the specific binder D-Ala-D-Ala) to evaluate the nonspecific binding. Each microtitre plate is then incubated for 2 h at room temperature in a humid covered box and washed eight times by emptying and filling with phosphate buffered saline solution pH 7.3 (0.05 M sodium phosphate, 0.15 M sodium chloride containing 0.05% (v/v) Tween ® 20) and shaken dry.

Purified rabbit antiserum against teicoplanin prepared according to the foregoing Examples 3 and 4 is then diluted 50 fold with phosphate buffered saline solution (0.05 M sodium phosphate, 0.15 M sodium chloride, pH 7.3, containing 3% (w/v) BSA, 0.05% (v/v) Tween ® 20 and 20% human serum).

100 Microliter of this solution is added to each well and allowed to react for 1 h at room temperature. After washing eight times with phosphate buffered saline pH 7.3 (0.05 M sodium phosphate, 0.15 M sodium chloride containing 0.05% (v/v) Tween ® 20), shaken dry as described above, 100 microliter of a 1 to 500 dilution in phosphate buffered saline containing 20% human serum of horseradish peroxidase conjugated goat-antiserum to rabbit IgGs (Bionetics Inc.) is added to each well and allowed to stand for 1 h at room temperature. Then each well is again washed with phosphate buffered saline pH 7.3 (0.05 M sodium phosphate, 0.15 M sodium chloride · containing 0.05% (v/v) Tween ® 20) and shaken dry as described above and a 200 microliter/well solution of a peroxidase chromogenic substrate (1 mg/ml o-phenylendiamine, 5 mM hydrogen peroxide in 0.1 M sodium citrate buffer, pH 5) is added to each well and the color allowed to develop for 30 min at room temperature. The color developing reaction is then stopped by adding 50 microliter/well of 4.5 M sulfuric acid and, after 15 min, the absorbance at 492 nm is measured in a Titertek Multiscan photometer (Flow Lab. Inc.). Binding curves are obtained by plotting the values of absorbance as a function of the concentration of teicoplanin on a semi-log paper. A dose-response curve is obtained with teicoplanin in the range from 0.01 microgram/ml to 1 microgram/ml and the concentration in the sample is determined by interpolation on the standard curve.

The repeated assay of solutions containing known amounts of teicoplanin by means of the above procedure indicates that this method satisfies the analytical requirements of sensitivity, precision and accuracy.

In the case exemplified above, the following results, which are valid also in general terms for any application of the method of the invention, are obtained:

Application range (on the basis of repeated experiments): from 10 ng/ml

Preferred application range from 10 ng/ml to 1 mg/l of teicoplanin.

Precision:

- within the assay: CV% equal to 10.5
- between the assays: CV% equal to 13.2

No binding was observed repeating the above method and using solutions of avoparcin, ristocetin, actaplanin, antibiotic A 35512 B, antibiotic A 41030, antibiotic A 47934 and vancomycin.

Substantially applying the same procedure described in Example 6 above to pus, bronchial expectorates and burned skin samples from patients treated with teicoplanin, the concentration of this antibiotic substance has been determined, after homogenizing the sample in the 20% serum buffered solution described in the above Example. The method maintained also on these samples the same level of sensitivity, accuracy and precision reported above.

Preparation of teicoplanin

Teicoplanin is prepared by cultivating the strain Actinoplanes teichomyceticus ATCC 31121 as disclosed in US-A-4,239,751 and purified by means of Sephadex ® column chromatography or other equivalent purification technique.

Claims

1. A method of determining an analyte which binds to the "molecular receptor" represented by a D-Alanyl-D-Alanine dipeptide or a D-Alanyl-D-Alanine carboxy terminal oligopeptide, which method comprises:

   a) causing a test solution to contact with a surface supporting a D-Alanyl-D-Alanine carboxy terminal oligopeptide conjugated with a suitable macromolecular carrier capable of adsorbing onto said surface;

   b) after rinsing, adding antibodies specifically directed against the substance to be detected,

   c) revealing said antibodies bound to the antigen on the stationary phase by means of a species-specific anti-IgG-antibody directed toward these bound antibodies (anti-antibody antiserum) coupled to a detectable marker.

2. A method according to claim 1 wherein the analyte is an antibiotic substance of the vancomycin class selected from vancomycin, teicoplanin, actaplanin, ristocetin, avoparcin, actinoidin, antibiotic LL-AM-374, antibiotic A 477, antibiotic OA 7653, antibiotic A 35512 B, antibiotic A 51568, antibiotic AAD 216, antibiotic A 41030, antibiotic A 47934 as well as an individual factor, derivative or aglycon thereof.

3. A method according to claim 1 wherein the analyte is teicoplanin or an individual factor, derivative or aglycon thereof.

4. A method according to claim 1 wherein the D-Alanyl-D-Alanine carboxy terminal oligopeptide is Epsilon-aminocaproyl-D-Alanyl-D-Alanine.

5. A method according to claim 1 wherein the macromolecular carrier is a protein.

6. A method according to claim 1 wherein the macromolecular carrier is albumin.

7. A method according to claim 1 wherein the supporting surface is provided by the wells of microtiter plates or test tubes, or plastic sheets.

8. A method according to claim 1 wherein the supporting surface is the wells of polyethylene or polystyrene microtiter plates.

9. A method as claimed in claim 1 wherein the specific antibody is a rabbit antibody and the species-specific anti-antibody antiserum is a goat antiserum to rabbit IgGs.

10. A method as claimed in claim 1 wherein the specific antibody is a monoclonal antibody which does not impair the binding of the analyte with the adsorbed "molecular receptor".

11. An analytical kit for use in a method as claimed in claim 1 which kit comprises:

a) an analytical support having a surface supporting a D-Alanyl-D-Alanine carboxy terminal oligopeptide conjugated with a suitable macromolecular carrier capable of adsorbing onto said surface;

b) an antibody preparation containing antibodies specifically directed against the analyte which is the substance to be detected

12. A kit as claimed in claim 11 which far includes a species-specific anti-antibody preparation containing antibodies specifically directed against the immunoglobulins of the animal species of origin of the "first" antibodies directed against the analyte and coupled with a detectable marker.

13. A kit as claimed in claim 11 or 12 wherein the D-Alanyl-D-Alanine carboxy terminal oligopeptide is Epsilon-aminocaproyl-D-Alanyl-D-Alanine.

14. A kit as claimed in claim 11 or 12 wherein the macromolecular carrier is a protein.

15. A kit as claimed in claim 11 or 12 Wherein the macromolecular carrier is albumin.

16. A kit as claimed in claim 11 or 12 wherein the analytical support is the wells of microtiter plates or test tubes, or plastic sheets.

## Revendications

1. Méthode de détermination d'un analyte qui se lie au "récepteur moléculaire" représenté par un dipeptide D-Alanyl-D-Alanine ou par un oligopeptide à terminaison D-Alanyl-D-Alanine carboxy, laquelle méthode comprend :

a) le fait de mettre une solution d'essai en contact avec une surface portant un oligopeptide à terminaison D-Alanyl-D-Alanine carboxy conjugué à un support macromoléculaire approprié capable de s'adsorber sur cette surface ;

b) après rinçage, l'addition d'anticorps spécifiquement dirigés contre la substance à détecter ;

c) la révélation de ces anticorps liés à l'antigène sur la phase stationnaire au moyen d'un anticorps anti-IgG spécifique de l'espèce dirigé contre ces anticorps liés (antisérum anti-anticorps) couplé à un marqueur détectable.

2. Méthode suivant la revendication 1, dans laquelle l'analyte est une substance antibiotique de la classe de la vancomycine, choisie parmi la vancomycine, la téicoplanine, l'actaplanine, la ristocétine, l'avoparcine, l'actinoïdine, l'antibiotique LL-AM-374, l'antibiotique A 477, l'antibiotique OA 7653, l'antibiotique A 35512 B, l'antibiotique A 51568, l'antibiotique AAD 216, l'antibiotique A 41030, l'antibiotique A 47934 ainsi qu'un facteur individuel, un dérivé ou un aglycone de celle-ci.

3. Méthode suivant la revendication 1, dans laquelle l'analyte est la téicoplanine ou un facteur individuel, un dérivé ou un aglycone de celle-ci.

4. Méthode suivant la revendication 1 dans laquelle l'oligopeptide à terminaison D-Alanyl-D-Alanine carboxy est la Epsilon-aminocaproyl-D-Alanyl-D-Alanine.

5. Méthode suivant la revendication 1, dans laquelle le support macromoléculaire est une protéine.

6. Méthode suivant la revendication 1, dans laquelle le support macromoléculaire est l'albumine.

7. Méthode suivant la revendication 1, dans laquelle les moyens de support sont constitués par les puits de plaques de microtitrage ou par des tubes à essai, ou par des feuilles de matière plastique.

8. Méthode suivant la revendication 1, dans laquelle les moyens de support sont les puits de plaques de microtitrage de polyéthylène ou de polystyrène.

9. Méthode suivant la revendication 1, dans laquelle l'anticorps spécifique est un anticorps de lapin et l'antisérum anti-anticorps spécifique de l'espèce est un antisérum de chèvre contre les IgGs de lapin.

10. Méthode suivant la revendication 1, dans laquelle l' anticorps spécifique est un anticorps monoclonal qui n'altère par la liaison de l'analyte avec le "récepteur moléculaire" adsorbé.

11. Nécessaire analytique pour l'utilisation dans une méthode telle que revendiquée dans la revendication 1, lequel nécessaire comprend :

a) un support analytique ayant une surface portant un oligopeptide à terminaison D-Alanyl-D-Alanine carboxy conjugué avec un support macromoléculaire approprié, capable de s'adsorber sur cette surface;

b) une préparation d'anticorps contenant des anticorps spécifiquement dirigés contre l'analyte qui est la substance à détecter.

12. Nécessaire suivant la revendication 11, qui contient en outre

une préparation anti-anticorps spécifique de l'espèce contenant des anticorps spécifiquement dirigés contre les immunoglobulines de l'espèce animale à l'origine des "premiers anticorps" dirigés contre l'analyte, et couplés avec un marqueur détectable.

13. Nécessaire suivant la revendication 11 ou 12, dans lequel l'oligopeptide à terminaison D-Alanyl-D-Alanine carboxy est la Epsilon-aminocaproyl-D-alanyl-D-Alanine.

14. Nécessaire suivant la revendication 11 ou 12, dans lequel le support macromoléculaire est une protéine.

15. Nécessaire suivant la revendication 11 ou 12, dans lequel le support macromoléculaire est l'albumine.

16. Nécessaire suivant la revendication 11 ou 12, dans lequel le support analytique est constitué par les puits de plaques de microtitrage ou des tubes à essai, ou des feuille de matière plastique.

**Ansprüche**

1. Verfahren zur Bestimmung eines zu analysierenden Stoffes, der an ein D-Alanyl-D-Alanin-Dipeptid oder ein carboxyterminales D-Alanyl-D-Alanin-Oligopeptid als "molekularen Rezeptor" bindet, wobei in dem Verfahren

a) eine zu untersuchende Lösung mit einer Oberfläche in Kontakt gebracht wird, die ein carboxyterminales, an einen geeigneten makromolekularen Träger, der an diese Oberfläche adsorbieren kann, gekoppeltes D-Alanyl-D-Alanin-Oligopeptid trägt;

b) nach Waschen spezifisch gegen den nachzuweisenden Stoff gerichtete Antikörper zugegeben werden;

c) die an das Antigen auf der festen Phase gebundenen Anti-körper durch einen gegen diese gebundenen Antikörper gerichteten artspezifischen anti-IgG-Antikörper (anti-Antikörper-Antiserum), der an einen nachweisbaren Marker gekoppelt ist, sichtbar gemacht werden.

2. Verfahren nach Anspruch 1, wobei der zu analysierende Stoff ein antibiotischer Stoff der Vancomycin-Klasse, der Vancomycin, Teicoplanin, Actaplanin, Ristocetin, Avoparcin, Actinoidin, Antibiotikum LL-AM-374, Antibiotikum A 477, Antibiotikum OA 7653, Antibiotikum A 35512 B, Antibiotikum A 51568, Antibiotikum AAD 216, Antibiotikum A 41030 oder Antibiotikum A 47934, sowie ein einzelner Faktor, ein Derivat oder Aglycon davon ist.

3. Verfahren nach Anspruch 1, wobei der zu analysierende Stoff Teicoplanin oder ein einzelner Faktor, ein Derivat oder Aglycon davon ist.

4. Verfahren nach Anspruch 1, wobei das carboxyterminale D-Alanyl-D-Alanin-Oligopeptid Epsilon-aminocaproyl-D-Alanyl-D-Alanin ist.

5. Verfahren nach Anspruch 1, wobei der makromolekulare Träger ein Protein ist.

6. Verfahren nach Anspruch 1, wobei der makromolekulare Träger Albumin ist.

7. Verfahren nach Anspruch 1, wobei die Trägeroberfläche Vertiefungen von Mikrotiterplatten oder Reaktionsgefäße oder Plastikbögen ist.

8. Verfahren nach Anspruch 1, wobei die Trägeroberfläche Vertiefungen von Polyethylen- oder Polystyren-Mikrotiterplatten ist.

9. Verfahren nach Anspruch 1, wobei der spezifische Antikörper ein Kaninchen-Antikörper und das artspezifische anti-Antikörper-Antiserum ein Ziegen-Antiserum gegen Kaninchen-IgGs ist,

10. Verfahren nach Anspruch 1, wobei der spezifische Antikörper ein monoclonaler Antikörper ist, der die Bindung des zu analysierenden Stoffes mit dem adsorbierten "molekularen Rezeptor" nicht behindert.

11. Analytischer Kit zur Verwendung in einem Verfahren nach Anspruch 1, wobei der Kit enthält:

a) eine analytische Trägersubstanz mit einer Oberfläche, die ein an einen geeigneten makromolekularen Träger, der an die Oberfläche adsorbieren kann, gekoppeltes carboxyterminales D-Alanyl-D-Alanin-Oligopeptid trägt;

b) eine Antikörperpräparation, enthaltend spezifisch gegen den zu analysierenden Stoff, der der nachzuweisende Stoff ist, gerichtete Antikörper.

12. Kit nach Anspruch 11, der weiterhin eine artspezifische anti-Antikörperpräparation einschließt, die Antikörper enthält, die spezifisch gegen die Immunglobuline der Tierart gerichtet sind, aus der die "ersten" gegen den zu analysierenden Stoff gerichteten und an einen nachweisbaren Marker gekoppelten Antikörper stammen.

13. Kit nach Anspruch 11 oder 12, wobei das carboxyterminale D-Alanyl-D-Alanin-Oligopeptid Epsilon-aminocaproyl-D-Alanyl-D-Alanin ist.

14. Kit nach Anspruch 11 oder 12, wobei der makromolekulare Träger ein Protein ist.

15. Kit nach Anspruch 11 oder 12, wobei der makromolekulare Träger Albumin ist.

16. Kit nach Anspruch 11 oder 12, wobei die analytische Trägersubstanz Vertiefungen von Mikrotiterplatten, Reaktionsgefäße oder Plastikbögen ist.